Europäisches Patentamt

European Patent Office

Office européen des brevets

(11) Numéro de publication: **0 317 387 B1**

## (12) FASCICULE DE BREVET EUROPEEN

(45) Date de publication de fascicule du brevet: **13.10.93**  (51) Int. Cl.5: **A61K 37/36, C07K 7/10**

(21) Numéro de dépôt: **88402745.9**

(22) Date de dépôt: **02.11.88**

(54) **Procédé pour augmenter la production laitière chez les mammifères et/ou accroître le poids de leurs nouveaux-nés à la naissance et améliorer la croissance post-natale.**

(30) Priorité: **04.11.87 FR 8715313**

(43) Date de publication de la demande:
**24.05.89 Bulletin 89/21**

(45) Mention de la délivrance du brevet:
**13.10.93 Bulletin 93/41**

(84) Etats contractants désignés:
**AT BE CH DE ES FR GB GR IT LI LU NL SE**

(56) Documents cités:
**EP-A- 0 137 689**
**FR-A- 2 594 832**

**Commonwealth Agriculture Bank, 86302113CAB 860198070; D. Petitclerc et al.**

**Commonwealth Agriculture Bank, 87355649 CAB 870107052; P. Dubreuil et al.**

**Biological Abstracts, no. 33122957, H. Lapierre et al.**

**Biological Abstracts, no. 33095009, 33095008, D. Petitclerc et al.**

(73) Titulaire: **INSTITUT NATIONAL DE LA RECHERCHE AGRONOMIOUE**
**145, rue de l'Université**
**F-75341 Paris Cédex 07(FR)**

(72) Inventeur: **Kann, Guy**
**48 Ouai Alphonse Le Gallo**
**F-92100 Boulogne(FR)**
Inventeur: **Martinet, Jack**
**18 rue d'Auffargis**
**F-78120 Vieille Eglise(FR)**

(74) Mandataire: **Phélip, Bruno et al**
**c/o Cabinet Harlé & Phélip**
**21, rue de La Rochefoucauld**
**F-75009 Paris (FR)**

## Description

La présente invention concerne l'amélioration de la production laitière chez les mammifères à l'exclusion de l'homme ainsi que l'augmentation du poids de leurs nouveaux-nés à la naissance et l'amélioration de leur croissance post-natale. L'invention s'applique notamment aux animaux des espèces ovine, caprine, bovine et porcine.

Concernant la production laitière, les effets de l'hormone de croissance bovine (désignée ci-après par l'abréviation "bGH"), obtenue par recombinaison génétique, sur la lactation chez les vaches laitières ont été étudiés par Bauman D.E. et al, dont les travaux ont été rapportés dans "J. Dairy Sci., 65 (suppl 1) 121 (Abst.), 1982". Depuis lors, de nombreux travaux sur les bovins ont confirmé l'intérêt d'augmenter la galactopoièse à la suite de traitements quotidiens de vaches latières par la bGH pendant des lactations entières (Baird L.S. et al., "J. Dairy Sci., 69 (supp 1) 118 (Abst.) 1986"; Bauman D.E. et al., "J. Dairy Sci., 68 1352-62 (1985)"; Chapula W. et Schneider P.L., "Proc. Univ. Guelph Nutr. Conf. pp 106-21 (1986)"; Hutchinson C.F. et al, ("J. Dairy Sci., 69 (supp 1) 152 (Abst.) (1986)"; Mollett T.A. et al., "J. Dairy Sci., 69 - (supp 1) 118 (Abst.) (1986)"; et Soderhol M.C.G. et al. (J. Dairy Sci., 69 (supp 1), 152 (Abst.) (1986").

Après analyse de ces résultats ajustés à un lait à 3,5% en matières grasses, on observe une production laitière augmentée de 16, 19 et 22% pour des traitements respectifs de 12,5, 25 et 50 mg de bGH par jour. De plus, l'augmentation de la prise alimentaire chez les vaches traitées n'est pas proportionnelle à l'amélioration de la productivité laitière, de sorte que l'efficacité alimentaire (exprimée en kilogramme de lait à 3,5% de matières grasses par kilogramme de matière sèche ingérée) est augmentée respectivement de 13,7, 16,8 et 15,3% chez des animaux recevant 12,5, 25 ou 50 mg de bGH/jour pendant une longue période.

Le facteur de stimulation de la sécrétion de l'hormone de croissance humaine a été isolé, caractérisé, et il est maintenant synthétisé de façon industrielle. Ce facteur sera désigné ci-après par l'abréviation "hGRF". Sa forme naturelle contient 44 acides aminés. On connaît également la forme comportant 29 acides aminés, qui est obtenue après délétion de la partie C terminale de la molécule et dont l'activité biologique est similaire à celle de la molécule intacte. Dans les deux cas, le GRF humain ne diffère des GRF bovin et ovin correspondants que par un très petit nombre d'acides aminés.

Par ailleurs, la réalisation d'une forme lente est plus facile à réaliser pour l'hGRF que pour l'hormone de croissance humaine ou hormone ante-hypophysaire humaine (désignée ci-après par l'abréviation "hGH", toujours à cause de la taille plus petite de la molécule et d'un nombre plus faible de sites actifs à protéger au cours de la réalisation d'un "implant".

L'hGRF est actuellement utilisé en médecine humaine, comme facteur de libération de l'hormone de l'hGH (Guillemin R., Bazeau P., Bohlen P., Esch F., Ling N., Wehrenberg W.B., "Growth hormone releasing factor from a human pancreatic tumor that caused aeromegaly, Science, Wash. D.C., 218, 585.7, 1982").

La demande de brevet FR-A-2594832 indique que la stimulation de l'anabolisme protéique due à l'hormone de croissance confère au GRF un avenir particulièrement prometteur pour favoriser la production carnée et lactée des animaux d'élevage. L'objet de cette demande consiste en des dérivés du facteur de libération de l'hormone de croissance (GRF), possédant des amino-acides modifiés, ces dérivés étant applicables pour favoriser la croissance d'un être humain, bovin, porcin ou ovin. Cependant, il n'y a, dans cette demande antérieure aucune indication particulière sur les modalités du traitement.

La même remarque peut être faite vis-à-vis de la demande de brevet EU 137689 qui concerne des analogues du GRF et leur obtention, ainsi qu'une composition pour accélérer la croissance des animaux à sang chaud (non compris l'homme), pour accélérer la croissance et/ou la production de lait des animaux des espèces bovine ou caprine, pour accélérer la croissance des animaux de l'espèce porcine et pour accélérer la croissance et/ou la production de lait des animaux de l'espèce ovine. Dans le cas de l'administration de ces substances aux animaux, l'activité desdites substances est passagère et cesse dès que le traitement est interrompu.

Les mêmes observations peuvent être faites à propos d'autres documents illustratifs de la technique antérieure, à savoir:

- Commonwealth Agriculture Bank 86302113 CAB 860198070; D. Petitclerc et al, Sanofi Recherche 1986 p. 343-358.
- Commonwealth Agriculture Bank 87355649 CAB 870107052; P. Dubreuil et al, Reproduction, nutrition, developpement Vol. 27 n° 28, 1987, p. 601-603,
- Biological Abstracts n° 33122957; H. Lapierre et al, Journal of Animal Science, 1987, vol. 65, n° suppl. 1, p. 429,
- Biological Abstracts n° 33095008 et 33095009; D. Petitclerc et al, Journal of Dairy Science, 1987, vol. 70, n° suppl. 1, p 178.

On constate ainsi qu'aucun des documents illustrant la technique antérieure ne divulgue le traitement de mammifères, y compris l'homme, par l'hGRF, pendant des périodes de temps courtes au cours de la gestation, l'effet produit étant durable et se prolongeant durant toute la lactation.

Le problème auquel la présente invention apporte une solution est notamment d'obtenir une amélioration des performances laitières par un traitement des animaux pendant des périodes de temps courtes au cours de la gestation, afin d'éviter le traitement quotidien, lequel représente un inconvénient aussi bien sur le plan économique que sur le plan pratique.

On a maintenant trouvé - et ceci constitue la base de la présente invention - que l'administration aux femelles de l'hGRF, de ses fragments actifs, ou des analogues correspondants, pendant la gestation et, de façon plus précise, pendant une courte durée, à un moment déterminé de la gestation, permettait de résoudre le problème précité. Les fragments actifs correspondent à des molécules d'hGRF tronquées, mais comportant l'activité. Ainsi, la forme à 29 acides aminés, qui a été indiquée ci-dessus et sera désignée ci-après par l'abréviation "hGRF (1-29)", constitue une forme intéressante de la famille des substances actives selon l'invention.

Quant aux analogues mentionnés également ci -dessus, de l'hGRF ou de ses fragments actifs, ils correspondent respectivement à l'hGRF et aux fragments actifs dans lesquels au moins un acide ami né a été substitué.

De plus au cours des travaux ayant conduit à la pré sente invention, on a trouvé que l'administration des principes actifs susindiqués, toujours pendant la gestation, mais à un autre moment de celle-ci et toujours sur une période relativement courte, conduisait à une augmentation du poids des nouveaux-nés, à la naissance et à une amélioration de leur croissance post-natale, par rapport aux animaux issus de mères non traitées. Le traitement cumulé pendant les deux périodes précitées a également été réalisé.

A cet avantage d' un traitement sur une courte durée ou sur de courtes durées s'ajoute le fait que les substances précitées, et en particulier, le hGRF (1-29) est une molécule spécifique, donc applicable à toutes les espèces, et qui fait partie des hormones protéiques donc entièrement dégradées par l'organisme.

Parmi les nombreuses applications particulières que l'on peut mentionner pour la présente invention on peut signaler que chez la truie, il en résulte une augmentation du poids des porcelets et une diminution de l mortalité par hypotrophie; l'obtention dune puberté plus précoce chez la brebis et la vache (on pourra avancer ainsi la date de mise à la reproduction).

La présente invention a donc d'abord pour objet un procédé pour augmenter la production laitière chez les mammifères à l'exclusion de l'homme et/ou pour accroître le poids de leurs nouveauxnés à la naissance et améliorer la croissance post-natale selon lequel on fournit à la femelle du mammifère, pendant la gestation, une quantité efficace d'hGRF ou de l'un de ses analogues, ou encore d'un fragment actif de l'hGRF ou d'un de ses analogues.

Dans un mode de réalisation préféré, on utilise l'hGRF sous sa forme comportant 29 acides aminés (GRF 1-29 humain) ou un analogue de celle-ci.

La présente invention peut être mise en oeuvre à l'aide d'une composition contenant, comme principe actif, l'hGRF ou l'un de ses analogues, ou encore un fragment actif de l'hGRF ou de l'un de ses analogues, ledit principe actif étant associé à un véhicule physiologiquement acceptable.

En particulier, cette composition se présente sous une forme injectable, en solution dans un soluté physiologique.

La quantité de principe actif à injecter varie selon les espèces. Dans le cas de la brebis, elle est, de préférence de l'ordre de 25 μg/kg de poids corporel/jour par la voie sous-cutanée. La composition sera donc présentée en conséquence.

Conformément à un mode de réalisation particulier, le principe actif de la composition peut être mis sous une forme permettant sa libération lente dans l'organisme.

L'invention a encore pour objet:

- un procédé pour augmenter la production laitière chez les mammifères à l'exclusion de l'homme, selon lequel on fournit à la femelle du mammifère, sur une période de l'ordre de 10 à 20 jours, à la fin du deuxième tiers de la gestation, une quantité efficace de l'hGRF ou de l'un de ses analogues, ou encore d' un fragment actif de l'hGRF ou d' un de ses analogues.

- un procédé pour accroître le poids des nouveaux-nés des mammifères à l'exclusion de l'homme à la naissance et améliorer la croissance post-natale, selon lequel on fournit à la femelle du mammifère, sur une période de l'ordre de 10 à 20 jours précédent immédiatement la parturition, une quantité efficace de l'hGRF ou de l'un de ses analogues, ou encore d'un fragment actif de l'hGRF ou d'un de ses analogues.

- un procédé pour augmenter la production laitière chez les mammifères à l'exclusion de l'homme et/ou pour accroître le poids de leurs nouveaux-nés à la naissance et améliorer la croissance post-natale,

selon lequel on fournit à la femelle du mammifère, sur une période de l'ordre de 10 à 20 jours, à la fin du deuxième tiers de la gestation, et sur une période de l'ordre de 10 à 20 jours précédant immédiatement la parturition, une quantité efficace de l'hGRF ou de l'un de ses ànalogues, ou encore d' un fragment actif de l'HGRF ou d'un de ses analogues.

L'invention est applicable tout particulièrement aux animaux des espèces ovine, caprine, bovine ou porcine.

Les essais rapportés ci-après illustrent l'invention et les résultats avantageux qu'elle procure.

A - EFFETS DE l'ADMINISTRATION D'hGRF (1-29) AUX BREBIS EN FIN DE GESTATION, SUR LA CROISSANCE FOETALE

1 - Protocole expérimental

On a réparti, en deux lots de 15 brebis chacun, 30 brebis gestantes se trouvant au 135ème jour de gestation, en tenant compte du nombre de foetus supposés présents dans les utérus et des performances laitières antérieures, toutes les brebis étant multipares.

Le lot A, ne recevant aucun traitement: sert de lot témoin. Le lot B reçoit, par voie sous-cutanée, 1,5 mg d'hGRF (1-29) (soit environ 20 μg par kg de poids corporel), de façon biquotidienne, à 8 heures et 14 heures, à partir du jour 137 de gestation jusqu'au jour de la mise bas (lequel a varié entre le jour 145 et le jour 147 selon les brebis). L'administration d'hGRF (1-29) est interrompue dès que la parturition survient. Chez tous les animaux, l'agnelage est induit par administration de fluoro-9 α trihydroxy-11 β , 17 α,21 méthyl-16 α prégnadiène-1,4 dione-3,20 (proposée sous la DCI de Dexaméthasone) à raison de 16 mg par voie intramusculaire à 20 heures au jour 144 de la gestation.

On a comparé les poids à la naissance des agneaux issus des mères des deux lots A et B.

2 - Résultats

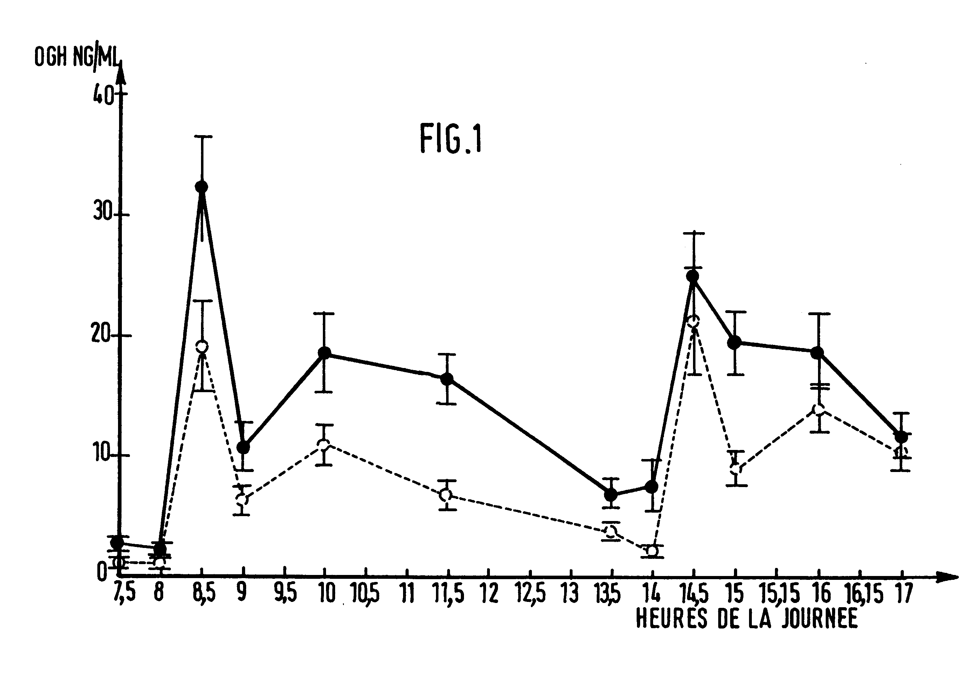

Les observations suivantes ont été faites:
. L'administration d'hGRF (1-29) provoque, aux doses utilisées, une décharge d' hormone de croissance (ci-après GH), de forte amplitude, dont la durée est d'environ 5 heures après l'injection sous-cutanée. Cette décharge est de même nature après l'injection du matin et après celle de l'après-midi. Par ailleurs, la réponse s'amplifie au fur et à mesure que l'on se rapproche du terme de la gestation. C'est ce que montre la figure 1 du dessins annexé qui représente le dosage hormonal OGH en fonction des heures de la journée (moyenne sur 15 brebis), au jour 138 et au jour 146 de la gestation.

Légende de la figure 1

o-----o    hGRF (1-29) administré comme indiqué à G138
•——•    hGRF (1-29) administré comme indiqué à G146.

Dans ces conditions, les animaux du lot B ont un taux de gH circulant pendant le jour qui est de 10 à 15 fois supérieur à celui du lot A.
. Les parturitions se sont déroulées dans les mêmes conditions pour les 2 lots, l'administration prépartum d'hGRF (1-29) ne modifiant pas les dates d'agnelage.
. Le poids à la naissance des agneaux issus de mères traitées (lot B, n = 20) est très significativement (p 0,003) plus élevé que celui des agneaux issus du lot témoin (lot A, n = 21), puisque les agneaux issus de brebis du lot B pèsent 4,17 ± 0,13 kg, pour 3,61 ± 0,14 kg pour les agneaux isus de mères du lot A.

Cette étude expérimentale a mis en évidence que le traitement des brebis par l'hGRF (1-29) dans l'immédiat prépartum induit une croissance in utero plus rapide des foetus.

B - UTILISATION D'hGRF (1-29) A LA FIN DU DEUXIEME TIERS DE LA GESTATION ET/OU EN FIN DE GESTATION CHEZ LES BREBIS: INCIDENCE SUR LA CROISSANCE FOETALE ET SUR LA CROISSANCE ULTERIEURE DES JEUNES BREBIS

1 - Protocole expérimental

On a réparti en quatre lots de 12 brebis chacun, 48 brebis gestantes multipares, en tenant compte des productions laitières antérieures et de la parité prévue (dosage OCS - Ovine Chorionic Somatotropin).

Le premier lot constitue le lot témoin, les brebis ne recevant aucun traitement.

Les trois autres lots reçoivent, par voie sous-cutanée, 1,5 mg d'hGRF (1-29), de façon biquotidienne;

. du jour 105 au jour 115 de la gestation pour le second lot, qui sera désigné dans ce qui suit comme étant le lot "GRF1";

. du jour 137 de gestation à la mise bas pour le troisième lot, qui sera désigné, dans ce qui suit, comme étant le lot "GRF2"; et

. du jour 105 au jour 115 de la gestation et du jour 137 de la gestation à la mise bas pour le quatrième lot, qui sera désigné, dans ce qui suit, comme étant le lot "GRF1 + 2".

Comme dans l'étude précédente, l'administration d'hGRF (1-29) est interrompue dès que la parturition survient et l'agnelage est induit par administration de Dexaméthasone au jour 145 de la gestation, dans les conditions indiquées précédemment.

Après la parturition, on a mesuré la production laitière des brebis de façon biquotidienne, et on a suivi la croissance des agneaux, lesquels ont reçu un allaitement artificiel fourni "ad libitum", les agneaux ne recevant aucun traitement adjuvant de croissance.

## 2 - Résultats

### a - Poids à la naissance

L'analyse a porté uniquement sur les agneaux doubles répartis comme suit:

Témoin n = 12
GRF2 n = 12
GRF1 n = 16
GRF1 + 2 n = 12

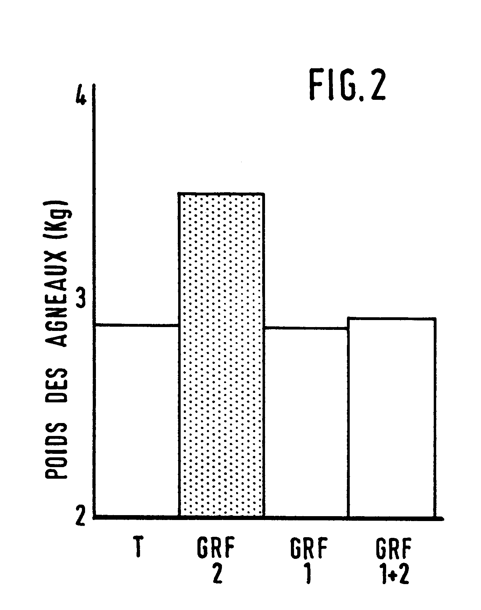

La figure 2 du dessins annexé, qui représente le diagramme du poids à la naissance des agneaux issus des quatre lots, montre que le traitement effectué induit, chez les brebis du lot GRF2, une croissance in utero plus grande des foetus, puisque la valeur moyenne du poids à la naissance est, pour ce lot, de 3,54 kg (p ≧ 0,007), alors qu' il n'est que de 2,91 kg pour les agneaux issus des mères "témoins".

La figure 2 montre également que les agneaux issus des lots GRF1 et GRF1 + 2 ont un poids de naissance sensiblement analogue à celui des agneaux issus des mères "témoins".

### b - Croissance des agneaux

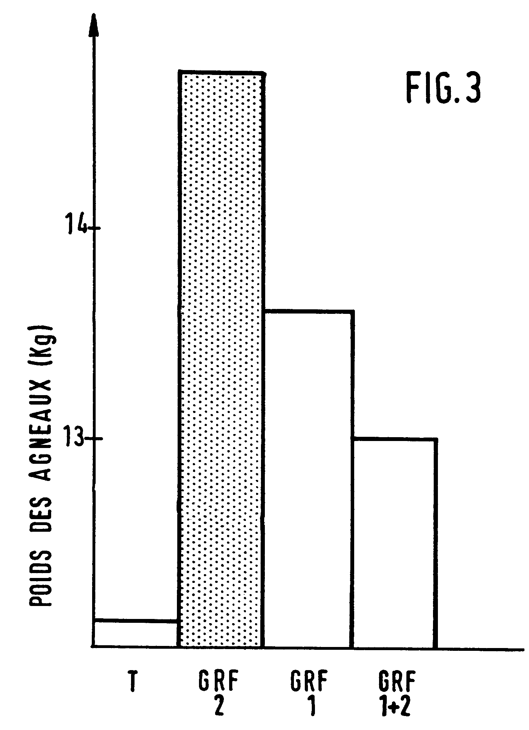

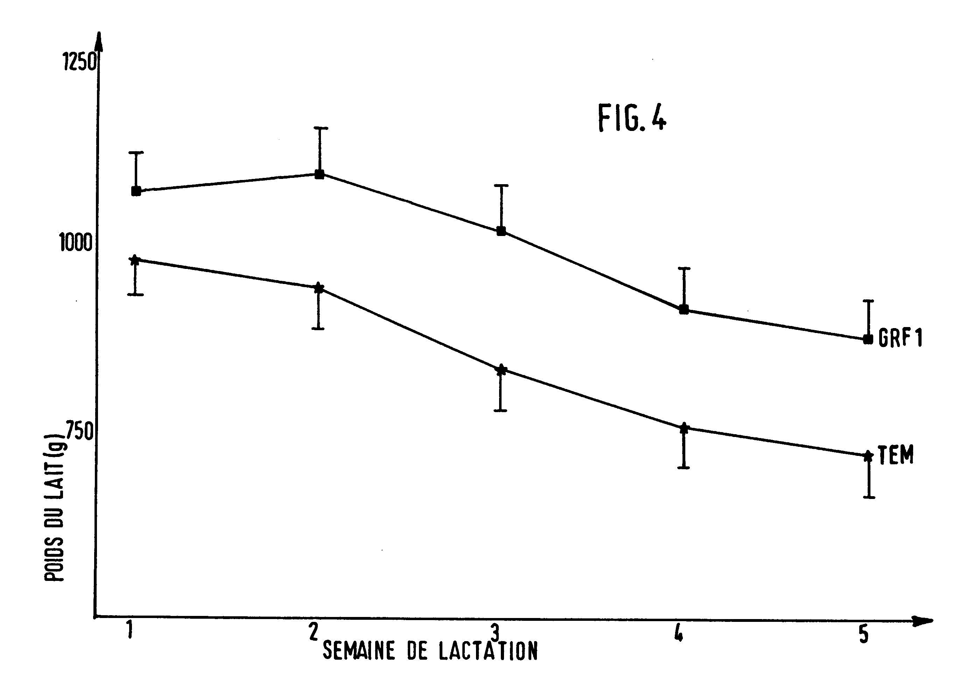

A l'âge de 30 jours, après analyse des variances prenant en covariable le poids à la naissance, on a observé une croissance plus importante de tous les agneaux issus des mères du lot GRF2. C'est ce que montre la figure 3 qui est une représentation analogue à celle de la figure 4, permettant de comparer les poids des agneaux issus des mères des quatre lots, à l'âge de 30 jours. Le Tableau 1 suivant complète cette figure 3.

TABLEAU 1

| Lot | Nombre d'agneaux | Poids des agneaux à 30 jours |
|---|---|---|
| Témoin | 7 | 12,12 ± 0,7kg |
| GRF2 | 12 | 14,73 ± 0,53kg (p>0,006) |
| GRF1 | 14 | 13,6 ± 0,5 kg (p>0,09) |
| GRF1 + 2 | 10 | 13 ± 0,66kg (ns) |

On constate que le traitement par hGRF (1-29) des mères entre les jours 137 et 147 de la gestation induit une croissance post-natale plus grande. Ainsi, la croissance de ces agneaux, même corrigée par la covariable que constitue le poids de naissance, est plus forte, puisqu'elle dépasse de 21% le poids des contrôles à 30jours.

Par ailleurs, quand les brebis ont été traitées par l'hGRF (1-29) entre les jours 105 et 115 de leur gestation, elles donnent naissance à des agneaux dont le poids de naissance est du même ordre que ceux des agneaux issus des mères "témoins", mais à 30 jours, la croissance de ces agneaux demeure encore supérieure, de l'ordre de 11%, à la croissance des agneaux issus des mères "témoins".

3 - Production laitière

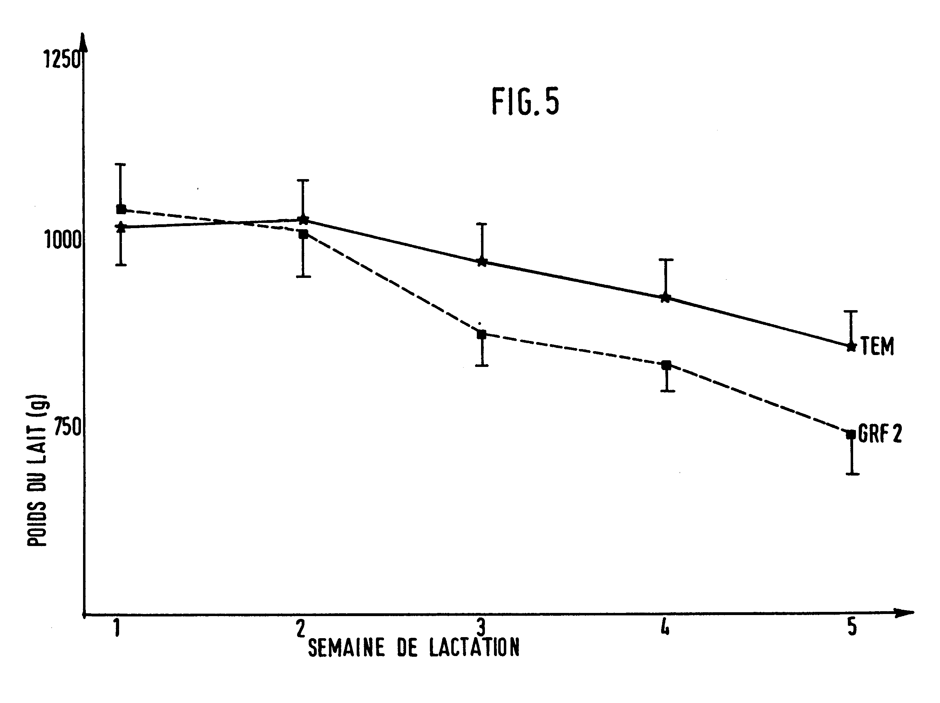

Après une analyse de variance, en prenant la production antérieure en covariable après correction tenant compte de la parité (nombre d'agneaux par gestation) lors de la lactation en cours et lors de la lactation de référence, on a comparé les résultats de la production laitière entre les lots témoins et les lots "GRF1" et "GRF2". A partir des résultats obtenus sur cinq semaines de lactation, on a tracé les courbes des figures 4 et 5, les données de base étant rapportées dans le Tableau 2 suivant.

## TABLEAU 2

### Production laitière

| Semaine de lactation | FIGURE 4 | |
|---|---|---|
| | Témoin | GRF1 |
| 1 | $980 \pm 70$ | $1075 \pm 50$ ns |
| 2 | $950 \pm 60$ | $1090 \pm 70$ ns |
| 3 | $845 \pm 50$ | $1025 \pm 60 \ p \geqslant 0,07$ |
| 4 | $760 \pm 45$ | $920 \pm 60 \ p \geqslant 0,1$ |
| 5 | $720 \pm 50$ | $880 \pm 50 \ p \geqslant 0,08$ |

| Semaine de lactation | FIGURE 5 | |
|---|---|---|
| | Témoin | GRF2 |
| 1 | $1020 \pm 50$ | $1050 \pm 60$ ns |
| 2 | $1025 \pm 60$ | $1010 \pm 50$ ns |
| 3 | $975 \pm 50$ | $855 \pm 50$ ns |
| 4 | $925 \pm 50$ | $840 \pm 40$ ns |
| 5 | $860 \pm 50$ | $745 \pm 60$ ns |

On constate que la production laitière des brebis traiteés entre les jours 105 et 115 de la gestation (lot "GRF1" est supérieure à celle des brebis "témoins" (plus de 23% lors de la cinquième semaine de lactation avec $p \geq 0,08$).

Tout se passe donc comme si le traitement par l'hGRF (1-29) entre les jours 105 et 115 de la gestation augmentait la mammogénèse chez les brebis traitées. Ces résultats confirment ceux obtenus avec l'induction de la lactation et montrent que la GH est bien une hormone importante dans le processus de la mammogénèse gravidique, agissant apparemment indépendamment de l'hormone placentaire OPL.

Le rôle de la GH comme facteur régulant l'orientation des "flux énergétiques" vers la mamelle ou vers l'utérus selon la période de stimulation par l'hGRF est mis en évidence, ce qui signifie qu'une brebis dont on a induit une forte mammogénèse ne donnera pas naissance à un agneau de plus grand poids, à l'inverse, celle que l'on stimule de façon à obtenir un agneau plus gros, aurait une production laitière diminuée.

6

**Revendications**

1. Procédé pour augmenter la production laitière chez les mammifères et/ou pour accroître le poids de leurs nouveaux-nés à la naissance et améliorer la croissance post-natale selon lequel on fournit à la femelle du mammifère à l'exclusion de l'homme, pendant la gestation, une quantité efficace d'hGRF, ou de l'un de ses analogues, ou encore d'un fragment actif de l'hGRF d'un de ses analogues.

2. Procédé pour augmenter la production laitière chez les mammifères à l'exclusion de l'homme, selon lequel on fournit à la femelle du mammifère, sur une période de l'ordre de 10 à 20 jours, à la fin du deuxième tiers de la gestation, une quantité suffisante de l'hGRF ou de l'un de ses analogues, ou encore d'un fragment de l'hGRF ou l'un de ses analogues.

3. Procédé pour accroître le poids des nouveaux-nés des mammifères à la naissance et améliorer la croissance post-natale, selon lequel on fournit à la femelle du mammifère à l'exclusion de l'homme, sur une période de l'ordre de 10 à 20 jours précédent immédiatement la parturition, une quantité efficace de l'hGRF ou l'un de ses analogues, ou encore d'un fragment actif de l'hGRF ou d'un de ses analogues.

4. Procédé pour augmenter la production laitière chez les mammifères à l'exclusion de l'homme et/ou pour accroître le poids de leurs nouveaux-nés à la naissance et améliorer la croissance post-natale, selon lequel on fournit à la femelle du mammifère à l'exclusion de l'homme, sur une période de l'ordre de 10 à 20 jours, à la fin du deuxième tiers de la gestation, et sur une période de l'ordre de 10 à 20 jours précédant immédiatement la parturition, une quantité efficace de l'hGRF ou de l'un de ses analogues, ou encore d'un fragment actif de l'hGRF ou l'un de ses analogues.

5. Procédé selon l'une quelconque des revendications 1 à 4, dans lequel on utilise le fragment actif d'hGRF qui comporte 29 acides aminés [hGRF (1-29)].

6. Procédé selon l'une quelconque des revendications 1 à 5, selon lequel on traite les animaux des espèces ovine, caprine, bovine ou porcine.

**Claims**

1. A method for increasing milk production in mammals , and/or for increasing the birth weight of their newborn and improving postnatal growth , according to which the female mammal excluding man is supplied during pregnancy with an effective amount of hGRF or of one of its analogs , or alternatively of an active fragment of hGRF or of one of its analogs .

2. A method for increasing milk production in mammals excluding man according to which the female mammal is supplied , over a period of the order of 10 to 20 days, on completion of two-thirds of the gestation period , with an effective amount of hGRF or of one of its analogs , or alternatively of an active fragment of hGRF or of one of its analogs .

3. A method for increasing the birth weight of new-born mammals , and improving postnatal growth , according to which the female mammal exclusing man is supplied , over a period of the order of 10 to 20 days immediately preceding parturition, with an effective amount of hGRF or of one of its analogs, or alternatively of an active fragment of hGRF or of one of is analogs.

4. A method for increasing milk production in mammals excluding man, and/or for increasing the birth weight of their newborn and improving postnatal growth, according to which the female mammal excluding man is supplied, over a period of the order of 10 to 20 days , on completion of two-thirds of the gestation period , and over a period of the order of 10 to 20 days immediately preceding parturition, with an effective amount of hGRF or of one of its analogs , or alternatively of an active fragment of GRF or of one of its analogs.

5. The method as claimed in any one of claims 1 to 4, in which the active fragment of hGRF which contains 29 amino acids [hGRF (1-29)]) is used.

**6.** The method as claimed in any one of claims 1 to 5, according to which animals of ovine , caprine, bovine or porcine species are treated.

**Patentansprüche**

**1.** Verfahren zur Steigerung der Milchproduktion bei Säugetieren und/oder zur Steigerung des Gewichts ihrer Neugeborenen bei der Geburt und zur Verbesserung des Wachstums nach der Geburt, gemäß dem man dem weiblichen Säugetier - mit Ausnahme des Menschen - während der Trächtigkeit eine wirksame Menge an hGRF oder eines seiner Analogen oder auch ein aktives Fragment eines der Analogen des hGRF zuführt.

**2.** Verfahren zur Steigerung der Milchproduktion bei Säugetieren - mit Ausnahme des Menschen - gemäß dem man dem weiblichen Säugetier während einer Zeitspanne von ungefähr 10 bis 20 Tagen, zu Ende des zweiten Drittels der Trächtigkeit, eine ausreichende Menge an hGRF oder eines seiner Analogen oder auch ein Fragment des hGRF oder eines seiner Analogen zuführt.

**3.** Verfahren zur Gewichtssteigerung der Neugeborenen von Säugetieren bei der Geburt und zur Verbesserung des Wachstums nach der Geburt, gemäß dem man dem weiblichen Säugetier - mit Ausnahme des Menschen - während einer Zeitspanne von ungefähr 10 bis 20 Tagen unmittelbar vor dem Werfen, eine wirksame Menge an hGRF oder eines seiner Analogen oder auch ein aktives Fragment des hGRF oder eines seiner Analogen, zuführt.

**4.** Verfahren zur Steigerung der Milchproduktion bei Säugetieren - mit Ausnahme des Menschen - und/oder zur Steigerung des Gewichts ihrer Neugeborenen bei der Geburt und zur Verbesserung des Wachstums nach der Geburt gemäß dem man dem weiblichen Säugetier - mit Ausnahme des Menschen - während einer Zeitspanne von ungefähr 10 bis 20 Tagen, zu Ende des zweiten Drittels der Trächtigkeit, und während eines Zeitraums von 10 bis 20 Tagen unmittelbar vor dem Werfen, eine wirksame Menge an hGRF oder eines seiner Analogen oder auch ein aktives Fragment des hGRF oder eines seiner Analogen, zuführt.

**5.** Verfahren gemäß einem der Ansprüche 1 bis 4, bei dem man das aktive Fragment des hGRF verwendet, welches 29 Aminosäuren [hGRF (1-29)] enthält.

**6.** Verfahren gemäß einem der Ansprüche 1 bis 5, gemäß dem man Tiere der Gattung Schaf, Rind oder Schwein behandelt.

FIG.1

EP 0 317 387 B1

FIG.2

FIG.3

FIG. 4

POIDS DU LAIT (g)

1250

1000

750

GRF 1

TEM

1    2    3    4    5

SEMAINE DE LACTATION

EP 0 317 387 B1

FIG.5